Europäisches Patentamt

European Patent Office

Office européen des brevets

(19)

(11) Veröffentlichungsnummer: **0 163 249 B1**

(12) **EUROPÄISCHE PATENTSCHRIFT**

(45) Veröffentlichungstag der Patentschrift: **31.07.91**

(51) Int. Cl.⁵: **C12N 15/26**, C12P 21/02, C07K 13/00, C12N 1/20

(21) Anmeldenummer: **85106279.4**

(22) Anmeldetag: **22.05.85**

(54) Gentechnologisches Verfahren zur Herstellung von Human-Interleukin-2 und Mittel zur Durchführung dieses Verfahrens.

(30) Priorität: **29.05.84 DE 3419995**

(43) Veröffentlichungstag der Anmeldung:
**04.12.85 Patentblatt 85/49**

(45) Bekanntmachung des Hinweises auf die Patenterteilung:
**31.07.91 Patentblatt 91/31**

(84) Benannte Vertragsstaaten:
**AT BE CH DE FR GB IT LI LU NL SE**

(56) Entgegenhaltungen:
**EP-A- 0 091 539**
**EP-A- 0 118 617**
**EP-A- 0 119 621**
**EP-A- 0 136 489**
**EP-A- 0 152 358**

(73) Patentinhaber: **HOECHST AKTIENGESELL-SCHAFT**
**Postfach 80 03 20**
**W-6230 Frankfurt am Main 80(DE)**

(72) Erfinder: **Engels, Joachim, Dr.**
**Feldbergstrasse 1**
**W-6242 Kronberg/Taunus(DE)**
Erfinder: **Uhlmann, Eugen, Dr.**
**Washington Street 287**
**Belmont Mass. 02178(US)**
Erfinder: **Wengenmayer, Friedrich, Dr.**
**Am Seyenbach 38**
**W-6238 Hofheim am Taunus(DE)**
Erfinder: **Müllner, Hubert, Dr.**
**Pestalozzistrasse 4**
**W-6233 Kelkheim (Taunus)(DE)**
Erfinder: **Winnacker, Ernst-Ludwig, Prof. Dr.**
**Dall'Armi-Strasse 41a**
**W-8000 München 19(DE)**
Erfinder: **Mertz, Ronald, Dr.**
**Ursulastrasse 5**
**W-8000 München 40(DE)**
Erfinder: **Okazaki, Hiroshi, Dr.**
**11-15, Yayoicho 6-chome Nakano-ku**
**Tokyo(JP)**

## Beschreibung

Die Erfindung ist in den Patentansprüchen definiert. Sie betrifft insbesondere ein Verfahren zur Herstellung von Human-Interleukin-2 und davon abgeleitete Polypeptide mit biologischer und immunologischer Aktivität von Human-Interleukin-2, chemisch synthetisierte Gene, die diese Peptide codieren sowie geeignete Vektorkonstruktionen und Wirtsorganismen zur Expression dieser Polypeptide.

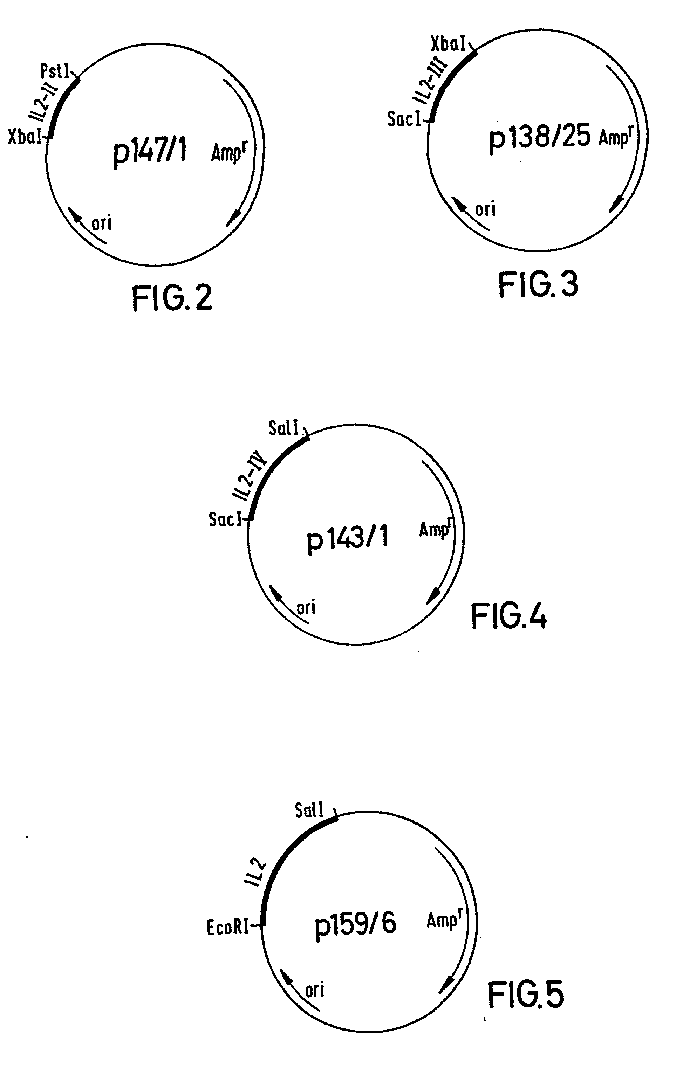

Human-Interleukin-2, im folgenden "IL-2", ist ein Polypeptid aus 133 Aminosäuren. Die DNA-Sequenz des menschlichen IL-2 sowie dessen gentechnologische Synthese sind in der Europäichen Patentanmeldung mit der Veröffentlichungsnummer EP 0 091 539 A1 beschrieben (Amino Acid Sequence II der Fig. 2b). Dieser Synthese liegt eine aus Säugetierzellen isolierte mRNA zugrunde, welche in cDNA überführt, in Vektoren eingebaut und in Wirtszellen, darunter auch E.coli, zur Expression gebracht wurde.

Bakterien, insbesondere E.coli, sind aus einer Reihe von Gründen bevorzugte Wirtszellen für die gentechnische Produktion von Polypeptiden. Gene eukaryotischer Zellen, die - wie in der genannten Patentanmeldung beschrieben - durch Reverse Transcriptase aus mRNA erhalten wurden, werden häufig nach Einbau in Plasmide und Transformation in Bakterien nur unbefriedigend exprimiert. Die Erfindung betrifft deshalb eine synthetische DNA-Sequenz, die für IL-2 codiert, welche besonders vorteilhaft in E.coli zur Expression eines Polypeptids mit IL-2-Aktivität führt. Ferner umfaßt die Erfindung solche DNA-Sequenzen, die gegen die synthetische DNA-Sequenz hybridieren und die sich aus dieser durch einfache oder multiple Substitionen, Deletionen oder Insertionen von Basen ableiten. Der Austausch, die Insertion oder die Deletion von Codons oder eine Kombination davon führt zu Peptiden mit einer oder mehreren ausgetauschten Aminosäure(n) oder zu längeren oder kürzeren IL-2-Derivaten, die ebenfalls Gegenstand der Erfindung sind. Diese Polypeptide mit der biologischen oder immunologischen Aktivität des IL-2 können in ihren Eigenschaften derart verändert sein, daß die Stabilität des Peptids erhöht ist, die Lipophilie des Polypeptids zugunsten einer besseren Löslichkeit verändert ist, die Applikation als Arzneimittel erleichtert ist oder aber die biologische Aktivität erhöht ist.

Der genetische Code ist bekanntlich "entartet", d.h. daß nur für zwei Aminosäuren eine einzige Nucleotid-Sequenz codiert, während den restlichen 18 genetisch codierbaren Aminosäuren 2 bis 6 Tripletts zuzuordnen sind. Von den hierdurch gegebenen Variationsmöglichkeiten machen jedoch die Wirtszellen unterschiedlicher Spezies nicht immer den gleichen Gebrauch. Für die Synthese der Gene besteht somit eine unübersehbare Vielfalt von Codon-Möglichkeiten.

Es wurde nun gefunden, daß die DNA-Sequenz I (Anhang), die für die gesamte Aminosäurensequenz 1-133 von IL-2 codiert, sowie die zur Synthese der Sequenz I benutzten DNA-Teilsequenzen (Sequenz II, Anhang, mit den Teilsequenzen IIa (IL 2-I) bis II d (IL 2-IV) besonders vorteilhaft für die gentechnologische Synthese von IL-2 sind. Am 5'-Ende des codierenden Stranges der DNA-Sequenz I befindet sich eine "überhängende" DNA-Sequenz, beispielsweise entsprechend der Restriktionsendonuclease Eco RI, am 3'-Ende des codierenden Stranges dagegen eine andere einzelsträngige, überhängende Sequenz, beispielsweise entsprechend dem Restriktionsenzym Sal I. Diese beiden unterschiedlichen Erkennungssequenzen gewährleisten die Insertion der DNA in Plasmide in der gewünschten Orientierung. Selbstverständlich können auch andere überstehende Sequenzen gewählt werden, die Schnittstellen im vorgesehenen Vektor entsprechen.

Diese Erkennungssequenzen erlauben nicht nur die Reisolierung der DNA aus einem Vektor durch Schneiden mit den entsprechenden Enzymen, beispielsweise Eco RI und SalI, sondern auch zahlreiche Modifikationen der DNA wie Entfernung der einzelsträngigen Bereiche, beispielsweise mit Mung Bean-Nuclease, völliges oder teilweises Auffüllen des kürzeren Endes, beispielsweise mit Kleenow-Polymerase, oder die Addition geeigneter Adapter oder Linker. Die überlappenden Enden sind daher außerordentlich vorteilhaft für den Einbau der erfindungsgemäßen DNA in Expressionsvektoren.

Zwischen diesen Erkennungssequenzen und den Codons für die Aminosäurefolge befindet sich am 5'-Ende des codierenden Stranges das Codon für die Aminosäure Methionin (das in der DNA-Sequenz I mit O beziffert ist). Alternativ hierzu kann eine Praesequenz (auch Signal- oder leader-Sequenz genannt), eines bakteriellen oder sonstigen wirtseigenen Proteins stehen (Übersichtsartikel: Perlman und Halvorson; J. Mol. Biol. 167 (1983), 391), welche die Sekretion des gewünschten Polypeptids aus dem Cytoplasma bewirkt und bei diesem Exkretionsprozeß von einer in der Wirtszelle natürlich vorkommenden Signal-Peptidase abgespalten wird. Am Ende des codierenden Stranges folgt bzw. folgen dann auf das für Threonin codierende Triplett 133 ein bzw. vorzugsweise zwei Stop-Triplett(s). Im Anhang ist die DNA-Sequenz I, welche die Nucleotide 9 bis 407 (Aminosäuren 1-133) umfaßt, im Zusammenhang mit der Nucleotidsequenz 1 bis 8 (überstehende Sequenz gemäß Eco RI und Met-Codon) und zwei Stop-Codons (Nucleotide 408 bis 413) sowie der überstehenden Sequenz gemäß SalI dargestellt. Die zwei Stop-Codons stellen eine bevorzugte Ausführungsform der Erfindung dar. Sie gewährleisten, daß selbst bei hoher Proteinsyntheserate

2

EP 0 163 249 B1

die Moleküle am gewünschten Ende "abgeschnitten" werden und keine "Fusionsproteine" gebildet werden.

Drei interne singuläre Schnittstellen für die Restriktionsenzyme Pst I, Xba I und Sac I (Nucleotide 69-74, 182-187 und 291-296 des codierenden Stranges der DNA-Sequenz I) ermöglichen die Subklonierung von vier Genfragmenten IL 2-I bis IL 2-IV, die in gut untersuchten Klonierungsvektoren, wie etwa pUC 12, eingebaut werden können. Zusätzlich wurden innerhalb des Strukturgens eine Reihe von weiteren singulären Erkennungssequenzen für Restriktionsenzyme eingebaut, die einerseits einen Zugang zu Teilsequenzen des IL-2 schaffen und andererseits die Durchführung von Variationen erlauben:

| Restriktionsenzym | Erkennungs- sequenz | | Position des ersten Nukleotids der Er- kennungssequenz (codierender Strang) |
|---|---|---|---|
| | 5' | 3' | |
| Aha II | GGCGCC | | 8 |
| Ava II | GGACC | | 65 |
| Ban I | GGCGCC | | 8 |
| Ban II | GAGCTC | | 291 |
| Bbv I | GCTGC | | 59 |
| Bst NI | CCTGG | | 221 |
| Dde I | CTCAG | | 226 |
| Fnu 4HI | GCTGC | | 59 |
| Hae II | GGCGCC | | 8 |
| Hha I | GCGC | | 9 |
| Hinf I | GACTC | | 35 |
| Hph I | TCACC | | 373 |
| Mlu I | ACGCGT | | 117 |
| Nar I | GGCGCC | | 8 |
| Pvu I | CGATCG | | 346 |
| Sau 96 I | GGACC | | 65 |
| Scr FI | CCTGG | | 221 |

Die DNA-Sequenz I läßt sich aus 38 Oligonucleotiden unterschiedlicher Länge aufbauen (siehe DNA-Sequenz II),indem diese zunächst chemisch synthetisiert und dann über "sticky ends" von 4 bis 9 Nucleotiden enzymatisch verknüpft werden.

Bei der DNA-Sequenz I wurde weiterhin berücksichtigt, daß bei denjenigen Aminosäuren, denen mehrere Codons zuzuordnen sind, diese nicht gleichwertig sind, sondern vielmehr in der jeweiligen Wirtszelle wie E. coli unterschiedliche Präferenzen zeigen. Weiterhin wurden palindromische Sequenzen auf ein Mindestmaß reduziert.

Die Genstruktur der DNA-Sequenz I ist somit leicht aus relativ kleinen Bausteinen zugänglich, ermöglicht die Subklonierung von vier Genfragmenten in gut bekannte Vektoren und erlaubt deren Kombination zum Gesamtgen. Die singulären Erkennungssequenzen für Restriktionsenzyme erleichtern außerordentlich die Bildung von Verlängerungen, Veränderungen und Verkürzungen des Proteinmoleküls.

Verlängerungen werden nach Umsetzung mit dem jeweiligen Restriktionsenzym durch Addition geeigneter, chemisch synthetisierter DNA-Moleküle erhalten. Veränderungen werden durch Herausschneiden einzelner Abschnitte der DNA mit geeigneten Restriktionsenzymen und Ersetzen durch andere, chemisch gewonnene DNA-Sequenzen erhalten. Verkürzungen können nach Spaltung mit dem jeweiligen Restriktions-

3

enzym durch Umsetzung mit Nucleasen erhalten werden.

Die biologische Aktivität der verlängerten, veränderten oder verkürzten Moleküle kann in einem biologischen Testsystem geprüft werden, beispielsweise durch Induktion des Zellwachstums einer T-Zell-Population, die von der Anwesenheit des IL-2 im Medium für die Vermehrung streng abhängig ist.

Der Einbau der synthetischen Gene bzw. Genfragmente in Klonierungsvektoren, beispielsweise in handelsübliche Plasmide wie pUC 12 bzw. andere allgemein zugängliche Plasmide wie ptac 11, ptrp H1 und pKK 177.3, erfolgt in an sich bekannter Weise. Auch können die chemisch synthetisierten Gene zuvor mit geeigneten chemisch synthetisierten Kontrollregionen versehen werden, die eine Expression der Proteine ermöglichen. Hierzu kann auf das Lehrbuch von Maniatis (Molecular Cloning, Maniatis et al., Cold Spring Harbor, 1982) verwiesen werden. Die Transformation der so erhaltenen Hybridplasmide in geeignete Wirtsorganismen, vorteilhaft E. coli, ist ebenfalls an sich bekannt und in dem vorstehend genannten Lehrbuch eingehend beschrieben.

Die erfindungsgemäß erhaltenen Genfragmente IL 2-I bis IL 2-IV, die damit erhaltenen Hybridplasmide und die transformierten Wirtsorganismen sind ebenfalls neu und Gegenstand der Erfindung. Dasselbe gilt für aus der DNA-Sequenz I abgewandelte neue DNA-Sequenzen. Weitere Ausgestaltungen der Erfindung sind in den Patentansprüchen niedergelegt.

In den folgenden Beispielen werden noch einige Ausgestaltungen der Erfindung im einzelnen erläutert, woraus sich die Vielzahl der möglichen Abwandlungen und Kombinationen für den Fachmann ergeben. Prozentangaben beziehen sich hierbei auf das Gewicht, wenn nichts anderes angegeben ist.

Beispiele

1. Chemische Synthese eines einzelsträngigen Oligonucleotids

Am Beispiel des Genbausteins Ia, der die Nucleotide 1-17 des codierenden Strangs umfaßt, wird die Synthese der Genbausteine erläutert. Nach bekannten Methoden (M.J. Gait et al., Nucleic Acids Res. 8 (1980) 1081-1096)) wird das am 3'-Ende stehende Nucleosid, im vorliegenden Falle also Cytidin (Nucleotid Nr. 17), an Kieselgel ($^{(R)}$FRACTOSIL, Firma Merck) über die 3'-Hydroxyfunktion covalent gebunden. Hierzu wird zunächst das Kieselgel unter Abspaltung von Ethanol mit 3-(Triethoxysilyl)-propylamin umgesetzt, wobei eine Si-O-Si-Bindung entsteht. Das Cytidin wird als $N^4$-Benzoyl-3'-O-succinoyl-5'-dimethoxytritylether in Gegenwart von Paranitrophenol und N,N'-Dicyclohexylcarbodiimid mit dem modifizierten Träger umgesetzt, wobei die freie Carboxygruppe der Succinoylgruppe den Aminorest der Propylaminogruppe acyliert.

In den folgenden Syntheseschritten wird die Basenkomponente als 5'-O-Dimethoxytrityl-nucleosid-3'-phosphorigsäuremonomethylester-dialkylamid oder -chlorid eingesetzt, wobei das Adenin als $N^6$-Benzoyl-Verbindung, das Cytosin als $N^4$-Benzoyl-Verbindung, das Guanin als $N^2$-Isobutyryl-Verbindung und das keine Aminogruppe enthaltende Thymin ohne Schutzgruppe vorliegen.

50 mg des polymeren Trägers, der 2 $\mu$mol Cytosin gebunden enthält, werden nacheinander mit den folgenden Agentien behandelt:

a) Nitromethan,

b) gesättigte Zinkbromidlösung in Nitromethan mit 1 % Wasser,

c) Methanol,

d) Tetrahydrofuran,

e) Acetonitril,

f) 40 $\mu$mol des entsprechenden Nucleosidphosphits und 200 $\mu$mol Tetrazol in 0,5 ml wasserfreiem Acetonitril (5 Minuten),

g) 20 % Acetanhydrid in Tetrahydrofuran mit 40 % Lutidin und 10 % Dimethylaminopyridin (2 Minuten),

h) Tetrahydrofuran,

i) Tetrahydrofuran mit 20 % Wasser und 40 % Lutidin,

j) 3 % Jod in Kollidin/Wasser/Tetrahydrofuran im Volumenverhältnis 5:4:1,

k) Tetrahydrofuran und

l) Methanol.

Unter "Phosphit" wird hierbei der Desoxyribose-3'-monophosphorigsäure-monomethylester verstanden, wobei die dritte Valenz durch Chlor oder eine tertiäre Aminogruppe, beispielsweise einen Morpholinorest, abgesättigt ist. Die Ausbeuten der einzelnen Syntheseschritte können jeweils nach der Detritylierungsreaktion b) spektrophotometrisch durch Messung der Absorption des Dimethoxytritylkations bei einer Wellenlänge von 496 nm bestimmt werden.

Nach abgeschlossener Synthese des Oligonucleotids werden die Methylphosphatschutzgruppen des Oligomers mit Hilfe von p-Thiokresol und Triethylamin abgespalten.

Anschließend wird durch 3-stündige Behandlung mit Ammoniak das Oligonucleotid vom festen Träger abgetrennt. Eine 2- bis 3-tägige Behandlung der Oligomeren mit konzentriertem Ammoniak spaltet die Aminoschutzgruppen der Basen quantitativ ab. Das so erhaltene Rohprodukt wird durch Hochdruckflüssigkeitschromatographie (HPLC) oder durch Polyacrylamid-Gelelektrophorese gereinigt.

Ganz entsprechend werden auch die übrigen Genbausteine Ib-IVj synthetisiert, deren Nucleotidfolge aus der DNA-Sequenz II hervorgeht.

2. Enzymatische Verknüpfung der einzelsträngigen Oligonucleotide zu den Genfragmenten IL 2-I bis IL 2-IV.

Zur Phosphorylierung der Oligonucleotide am 5'-Terminus wurde je 1 nmol der Oligonucleotide Ia und Ib mit 5 nmol Adenosintriphosphat mit vier Einheiten T4-Polynucleotid-Kinase in 20 $\mu$l 50 mM Tris-HCl-Puffer (pH 7,6), 10 mM Magnesiumchlorid und 10 mM Dithiothreitol (DTT) 30 Minuten bei 37°C behandelt (C.C. Richardson, Progress in Nucl. Acids Res. 2 (1972) 825). Das Enzym wird durch fünfminütiges Erhitzen auf 95°C desaktiviert. Anschließend werden die Oligonucleotide Ia und Ib gegeneinander hybridisiert, indem man sie in wäßriger Lösung 2 Minuten auf 95°C erhitzt und dann langsam auf 5°C abkühlt.

Analog werden die Oligonucleotide Ic und Id sowie Ie und If phosphoryliert und paarweise hybridisiert. Für das Subfragment IL 2-II werden die Oligonucleotide IIa mit IIb usw. bis IIi mit IIj, für das Subfragment IL 2-III die Oligomeren IIIa mit IIIb usw. bis IIIk mit IIIl und für das Subfragment IL 2-IV die Oligomeren IVa mit IVb usw. bis IVi mit IVj phosphoryliert und paarweise hybridisiert.

Die so erhaltenen drei Oligonucleotidpaare für das Genfragment IL 2-I, die fünf Oligonucleotidpaare für die Genfragmente IL 2-II und IL 2-IV sowie die sechs Oligonucleotidpaare für das Genfragment IL 2-III werden jeweils wie folgt ligiert:

Die doppelsträngigen Nucleotide werden vereinigt und in jeweils 40 $\mu$l 50 mM Tris-HCl-Puffer, 20 mM Magnesiumchlorid und 10 mM DTT mit Hilfe von 100 Einheiten T4-DNA-Ligase bei 15°C im Laufe von 16 Stunden ligiert.

Die Reinigung der Genfragmente IL 2-I bis IL 2-IV erfolgt durch Gelelektrophorese auf einem 10%igen Polyacrylamidgel (ohne Harnstoffzusatz, 20 • 40 cm, 1 mm Dicke), wobei als Markersubstanz ØX 174 DNA (Fa. BRL), geschnitten mit Hinf I, oder pBR 322, geschnitten mit Hae III, diente.

3. Herstellung von Hybridplasmiden, die die Genfragmente IL 2-I bis IL 2-IV enthalten

a) Einbau des Genfragmentes IL 2-I in pUC 12

Das Plasmid pUC 12 entspricht dem Plasmid pUC 8 (Vieira et al., Gene 19 (1982) 259-268; Messing et al., ibid. 269-276), enthält allerdings einen etwas vergrößerten Polylinker mit den zusätzlichen Restriktionsstellen für die Enzyme XbaI und SacI (Norrander et al., Gene 26 (1983) 101-106). Das Plasmid wird mit den Enzymen EcoRI und PstI inkubiert. Hierdurch wird ein etwa 40 Basenpaare großes Polynucleotid aus dem Plasmid herausgeschnitten. Die Abtrennung dieses Fragments vom geöffneten Plasmid gelingt entweder durch Chromatographie an [R]SEPHADEX G50 oder durch Elektrophorese an 1,5 %iger Agarose in bekannter Weise (Maniatis). Auf eine Abtrennung des Fragments kann auch verzichtet werden, da Plasmide mit der Insertion von IL 2-I leicht durch Ausplattieren erkannt werden können (wie im folgenden ausgeführt wird):

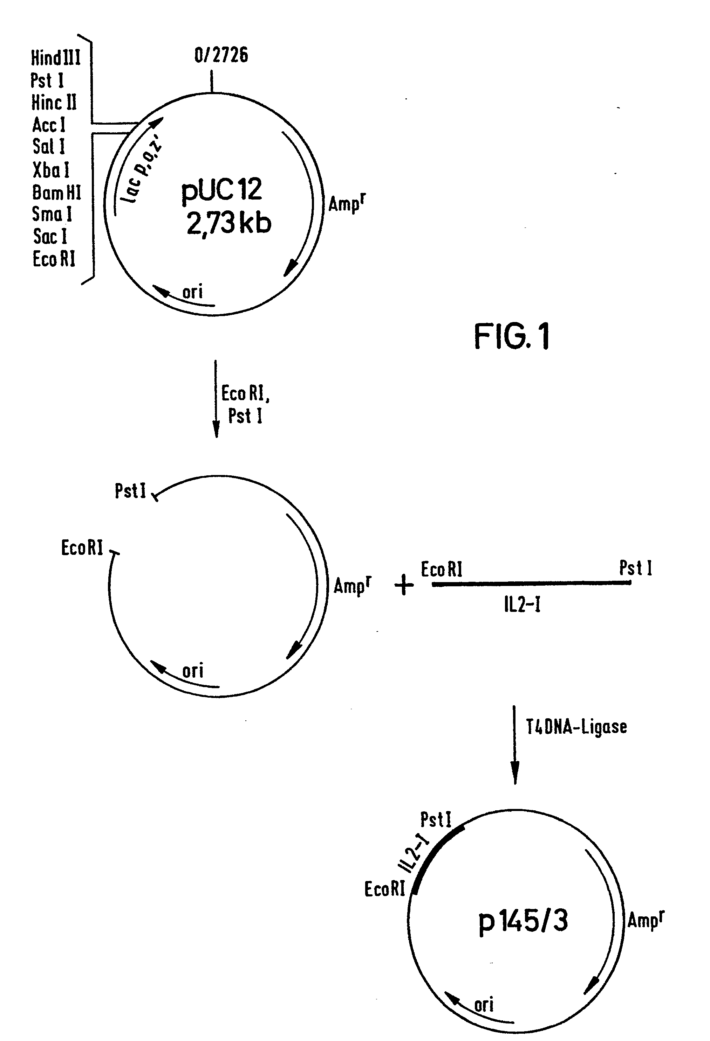

In das geöffnete Plasmid wird das Fragment IL 2-I wie folgt enzymatisch eingesetzt, wobei das Plasmid p 145/3 (Figur 1) gebildet wird:

Die DNA wird in einer Mischung aus 50 mM Tris (pH 7,6), 5 mM ATP, 5 mM Dithiothreitol (DTT), 5mM MgCl$_2$ und ca. 100 Einheiten T4 DNA-Ligase 16 Stunden bei 12°C ligiert. Anschließend wird das Plasmid in E.coli K 12 (JM 103), kompetent gemacht mit 70 mM CaCl$_2$, transformiert.

Bakterien, die das Plasmid p 145/3 enthalten, können auf Agar-Platten mit 50 $\mu$g/ml Ampicillin, 1 mM Isopropyl-Thiogalactosid (IPTG) und 2 % 5-Brom-4-chlor-3-indolyl-$\beta$-D-galactosid (X-Gal) als "weiße" Kolonie entdeckt werden. Eventuell noch unverändertes oder nicht vollständig geschnittenes Plasmid pUC 12 verursacht "blaue" Bakterienkolonien. Etwa fünf der weißen Bakterienklone werden kultiviert und die enthaltenen Plasmide in bekannter Weise (Maniatis) isoliert.

Die Größe der Insertion wird durch Inkubation mit den Restriktionsenzymen PstI und EcoRI und anschließende Elektrophorese auf 10%igem Polyacrylamidgelen überprüft. Hierauf erfolgt eine Sequenzierung der Insertion nach Maxam und Gilbert (Methods Enzymol. 65 (1980) 499) oder Sanger und Coulson (J. Mol. Biol. 94 (1975) 441).

b) Einbau des Genfragments IL 2-II in pUC 12

Analog zu a) wird das Plasmid pUC 12 mit den Restriktionsenzymen Pst I und Xba I umgesetzt und, ggf. nach Abtrennung des entstandenen Oligonucleotids, das Fragment IL 2-II enzymatisch eingebaut. Es entsteht das Plasmid p 147/1 (Figur 2).

c) Einbau des Genfragments IL 2-III in pUC 12

Analog zu a) wird das Plasmid pUC 12 mit den Restriktionsenzymen Xba I und Sac I umgesetzt und das Fragment IL 2-III enzymatisch eingebaut. Es entsteht das Plasmid p 138/25 (Figur 3).

d) Einbau des Genfragments IL 2-IV in pUC 12

Analog zu a) wird das Plasmid pUC 12 mit den Restriktionsenzymen Sac I und Sal I geschnitten und das Fragment IL 2-IV enzymatisch eingebaut. Es entsteht das Plasmid p 143/1 (Figur 4).

4. Verknüpfung der Genfragmente

Nach Vermehrung der Plasmide p 145/3, p 147/1, p 138/25 und p 143/1 und Bestätigung der Sequenz werden die Subfragmente IL 2-I bis IL 2-IV erneut mit den entsprechenden Restriktionsenzymen herausgeschnitten und durch Elektrophorese auf Polyacrylamid abgetrennt. Nach Eintauchen der Gele in eine wäßrige Lösung von Ethidiumbromid werden die Banden unter UV-Licht identifiziert, herausgeschnitten und die DNA in bekannter Weise eluiert (Maniatis).

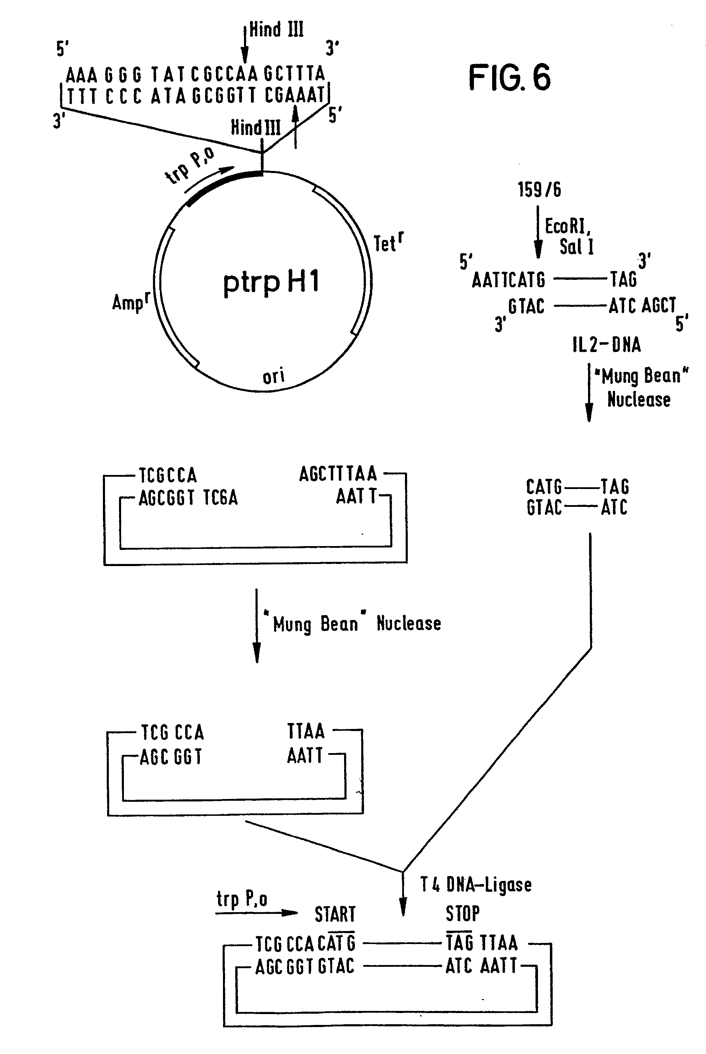

Die Subfragmente IL 2-I bis IL 2-IV werden wie unter 3 a) beschrieben enzymatisch verknüpft und in das durch Umsetzen mit den Restriktionsenzymen Eco RI und Sal I geöffnete Plasmid pUC 12 eingebaut. Man erhält das Hybridplasmid p 159/6 (Figur 5), dessen Sequenz nochmals durch Analyse bestätigt wird.

5. Konstruktion von Hybridplasmiden für die Expression der DNA-Sequenz I

a) Einbau in pKK 177.3

Das Expressionsplasmid pKK 177.3 (Plasmid ptac 11, Amman et al., Gene 25 (1983) 167, bei dem in die Eco RI-Erkennungsstelle synthetisch eine Sequenz eingebaut wurde, die eine Sal I-Schnittstelle enthält) wird mit den Restriktionsenzymen Eco RI und Sal I geöffnet. Aus dem Plasmid p 159/6 (Figur 5) wird die DNA-Sequenz I mit den Restriktionsenzymen Eco RI und Sal I herausgeschnitten und auf Polyacrylamid oder 2%ige niedrig-schmelzende (low melting) Agarose gegeben, von der Plasmid-DNA abgetrennt und die Insertion wiedergewonnen (Maniatis). Durch Ligation des aufgeschnittenen Plasmids pKK 177.3 mit der DNA-Sequenz I wird ein Hybridplasmid geschaffen, bei dem der Insertion eine Epressions- bzw. Regulationsregion vorgeschaltet ist. Nach Zugabe eines geeigneten Induktors wie IPTG wird eine mRNA gebildet, die zur Expression des Polypeptids entsprechend der DNA-Sequenz I führt.

b) Einbau in p trp H1

Das Expressionsplasmid p trp H1 (Amann et al., Gene 25 (1983) 167-178) enthält die Kontrollelemente des Trp-Operons (Promotor, Operator), gefolgt von einer Hind III-Restriktionsstelle (Fig. 6).

Nach Isolieren der DNA-Sequenz I aus p 159/6 werden die überstehenden Enden mit Mung Bean Nuclease nach Angaben des Herstellers (PL Biochemicals) abgebaut. p trp H1 wird mit Hind III geöffnet und die überstehenden Enden ebenfalls mit Mung Bean Nuclease entfernt. Die DNA-Sequenz I wird dann "stumpfendig" mit T 4 DNA-Ligase in das geöffnete Plasmid eingebaut (Fig. 6).

Durch das Triplett ATG für Methionin in Position 0 wird der Start für die Proteinsynthese festgelegt. Die Expression wird durch Abwesenheit von Tryptophan und/oder Anwesenheit von Indolylacrylessigsäure induziert.

6. Darstellung von Modifikationen

a) Verkürzungen am C-terminalen Ende des Proteins

Zur Darstellung von verkürzten Proteinmolekülen mit biologischer Aktivität des IL-2 wurde das Plasmid p 159/6 mit dem Restriktonsenzym Sal I umgesetzt und anschließend in an sich bekannter Weise mit Exonuclease III und S 1-Nuclease inkubiert. Nach Umsetzung mit Eco RI wurden nun Teilsequenzen des IL-2 erhalten, die an einem Ende die überlappenden Sequenzen für Eco RI tragen und am anderen Ende stumpfendig sind. Nach Addition einer chemisch synthetisierten DNA, die an einem Ende stumpfendig ist und hier als erstes Codon ein Stop-Codon aufweist, am anderen Ende dagegen den Überhang einer Sal I-Sequenz trägt, kann diese verkürzte DNA-Sequenz in die gleichen Expressionsvektoren eingebaut werden.

b) Verkürzungen am N-terminalen Ende des Proteins.

Zur Konstruktion eines Hybridplasmids, das die DNA-Sequenz für die Expression von Des-[Ala$^1$]-IL-2 enthält, wird aus dem Plasmid p 159/6 nach an sich bekannten Methoden mit den Restriktionsenzymen Eco RI und Hind III die DNA-Sequenz I inklusive Polylinkerteil des Plasmids herausgeschnitten, elektrophoretisch vom Restplasmid abgetrennt und mit dem Restriktionsenzym Aha II nachgeschnitten.

Das isolierte Aha II-Hind III-Fragment wird wie in Beispiel 5 b) beschrieben "stumpfendig" gemacht und dann mit Sal I nachgeschnitten. Mit Hilfe des folgenden Adaptors

$$5' \quad AA \ TTC \ ATG \quad 3'$$
$$3' \qquad \quad G \ TAC \quad 5'$$

erhält man nach Ligation mit dem mit Eco RI und Sal I geöffneten Plasmid pKK 177.3 ein neues Hybridplasmid, das das Gen zur Herstellung von Des-[Ala¹]-IL-2 enthält.

7. Transformation der Hybridplasmide.

Kompetente E. coli-Zellen werden mit 0,1 bis 1 µg der Hybridplasmide, die die Sequenz I oder Derivate davon enthalten, transformiert und auf Ampicillin enthaltende Agarplatten plattiert. Anschließend lassen sich Klone, die die korrekt integrierte IL-2-Gensequenz oder Derivate davon in den entsprechenden Plasmiden enthalten, durch DNA-Schnellaufarbeitung identifizieren (Maniatis a.a.O.).

8. Expression der IL-2-Aktivität aufweisenden Polypeptide

Nach Transformation der Hybridplasmide mit der DNA-Sequenz I oder Derivaten davon in E. coli wird ein Polypeptid exprimiert, das außer der IL-2-Aminosäuresequenz bzw. abgewandelte Sequenzen am Aminoterminus noch eine zusätzliche Methionylgruppe trägt.

9. Aufarbeitung und Reinigung

Die zur gewünschten optischen Dichte kultivierten Bakterienstämme werden mit einem geeigneten Induktor, beispielsweise IPTG, hinreichend lange, beispielsweise 2-4 Stunden, inkubiert. Anschließend werden die Zellen mit 0,1 % Kresol und 0,1 mM Benzylsulfonylfluorid abgetötet. Auf einem SDS-PAA-Gel ist nach der Induktion eine Proteinbande mit einem Molgewicht von etwa 15 000 Dalton festzustellen. Nach Zentrifugieren oder Filtrieren wird die Zellmasse in einer Pufferlösung (50 mM Tris, 50 mM EDTA, pH 7,5) aufgenommen und mechanisch aufgeschlossen, beispielsweise mit einer French-Presse bzw. (R)DYNO-Mühle (Fa. Willy Bachofer, Basel), worauf die unlöslichen Bestandteile abzentrifugiert werden.

Ein Teil des IL-2-Proteins verbleibt nach dem Zellaufschluß im Rückstand und kann mit 8 M Harnstoff, 6 M Guanidinium-Hydrochlorid, Pufferlösungen mit Detergentien ionischer oder nicht-ionischer Art und ähnlichen Lösemitteln solubilisiert werden. Aus diesen Lösungen wird das die IL-2-Aktivität enthaltende Protein nach üblichen Verfahren gereinigt. Geeignet sind Ionenaustauscher-, Adsorptions-, Gelfiltrationssäulen oder Affinitätschromatographie an Antikörpersäulen. Durch Natriumdodecylsulfat-Acrylamidgel- oder HPLC-Analytik werden Anreicherung und Reinheit des Produktes kontrolliert.

Zur biologischen Charakterisierung des IL-2-Proteins werden T-Zellen verwendet, die strikt abhängig von der Anwesenheit von IL-2 im Medium sind. Die Zellteilungsrate solcher Zell-Linien ist in gewissen Grenzen proportional zur IL-2-Konzentration des Mediums, die daher über die Aufnahme von ³H-Thymidin aus dem Medium festgestellt werden kann.

7

Tabelle 1

## DNA-Sequenz I

| Triplett Nr. | | | | | | | 0 | 1 | 2 |
|---|---|---|---|---|---|---|---|---|---|
| Aminosäure | | | | | | | Met | Ala | Pro |
| Nucleotid Nr. | | | | 1 | | | | 10 | |
| Cod. Strang | | | 5' AA | TTC | | ATG | GCG | CCG |
| nicht cod. Strang | | | 3' | G | | TAC | CGC | GGC |

| 3 | 4 | 5 | 6 | 7 | 8 | 9 | 10 | 11 | 12 |
|---|---|---|---|---|---|---|---|---|---|
| Thr | Ser | Ser | Ser | Thr | Lys | Lys | Thr | Gln | Leu |
| | 20 | | | | 30 | | | 40 | |
| ACC | TCT | TCT | TCT | ACC | AAA | AAG | ACT | CAA | CTG |
| TGG | AGA | AGA | AGA | TGG | TTT | TTC | TGA | GTT | GAC |

| 13 | 14 | 15 | 16 | 17 | 18 | 19 | 20 | 21 | 22 |
|---|---|---|---|---|---|---|---|---|---|
| Gln | Leu | Glu | His | Leu | Leu | Leu | Asp | Leu | Gln |
| | 50 | | | | 60 | | | 70 | |
| CAA | CTG | GAA | CAC | CTG | CTG | CTG | GAC | CTG | CAG |
| GTT | GAC | CTT | GTG | GAC | GAC | GAC | CTG | GAC | GTC |

| 23 | 24 | 25 | 26 | 27 | 28 | 29 | 30 | 31 | 32 |
|---|---|---|---|---|---|---|---|---|---|
| Met | Ile | Leu | Asn | Gly | Ile | Asn | Asn | Tyr | Lys |
| | 80 | | | | 90 | | | 100 | |
| ATG | ATC | CTG | AAC | GGT | ATC | AAC | AAC | TAC | AAA |
| TAC | TAG | GAC | TTG | CCA | TAG | TTG | TTG | ATG | TTT |

| 33 | 34 | 35 | 36 | 37 | 38 | 39 | 40 | 41 | 42 |
|---|---|---|---|---|---|---|---|---|---|
| Asn | Pro | Lys | Leu | Thr | Arg | Met | Leu | Thr | Phe |
| | 110 | | | | 120 | | | 130 | |
| AAC | CCG | AAA | CTG | ACG | CGT | ATG | CTG | ACC | TTC |
| TTG | GGC | TTT | GAC | TGC | GCA | TAC | GAC | TGG | AAG |

8

| 43 | 44 | 45 | 46 | 47 | 48 | 49 | 50 | 51 | 52 |
|---|---|---|---|---|---|---|---|---|---|
| Lys | Phe | Tyr | Met | Pro | Lys | Lys | Ala | Thr | Glu |
| | 140 | | | | 150 | | | 160 | |
| AAA | TTC | TAC | ATG | CCG | AAA | AAA | GCT | ACC | GAA |
| TTT | AAG | ATG | TAC | GGC | TTT | TTT | CGA | TGG | CTT |

| 53 | 54 | 55 | 56 | 57 | 58 | 59 | 60 | 61 | 62 |
|---|---|---|---|---|---|---|---|---|---|
| Leu | Lys | His | Leu | Gln | Cys | Leu | Glu | Glu | Glu |
| | 170 | | | | 180 | | | 190 | |
| CTG | AAA | CAC | CTC | CAG | TGT | CTA | GAA | GAA | GAG |
| GAC | TTT | GTG | GAG | GTC | ACA | GAT | CTT | CTT | CTC |

| 63 | 64 | 65 | 66 | 67 | 68 | 69 | 70 | 71 | 72 |
|---|---|---|---|---|---|---|---|---|---|
| Leu | Lys | Pro | Leu | Glu | Glu | Val | Leu | Asn | Leu |
| | 200 | | | | 210 | | | 220 | |
| CTG | AAA | CCG | CTG | GAG | GAA | GTT | CTG | AAC | CTG |
| GAC | TTT | GGC | GAC | CTC | CTT | CAA | GAC | TTG | GAC |

| 73 | 74 | 75 | 76 | 77 | 78 | 79 | 80 | 81 | 82 |
|---|---|---|---|---|---|---|---|---|---|
| Ala | Gln | Ser | Lys | Asn | Phe | His | Leu | Arg | Pro |
| | 230 | | | | 240 | | | 250 | |
| GCT | CAG | TCT | AAA | AAT | TTC | CAC | CTG | CGT | CCG |
| CGA | GTC | AGA | TTT | TTA | AAG | GTG | GAC | GCA | GGC |

| 83 | 84 | 85 | 86 | 87 | 88 | 89 | 90 | 91 | 92 |
|---|---|---|---|---|---|---|---|---|---|
| Arg | Asp | Leu | Ile | Ser | Asn | Ile | Asn | Val | Ile |
| | 260 | | | | 270 | | | 280 | |
| CGT | GAC | CTG | ATC | TCT | AAC | ATC | AAC | GTT | ATC |
| GCA | CTG | GAC | TAG | AGA | TTG | TAG | TTG | CAA | TAG |

| 93 | 94 | 95 | 96 | 97 | 98 | 99 | 100 | 101 | 102 |
|---|---|---|---|---|---|---|---|---|---|
| Val | Leu | Glu | Leu | Lys | Gly | Ser | Glu | Thr | Thr |
| | 290 | | | | 300 | | | 310 | |
| GTT | CTG | GAG | CTC | AAA | GGT | TCT | GAA | ACC | ACG |
| CAA | GAC | CTC | GAG | TTT | CCA | AGA | CTT | TGG | TGC |

| 103 | 104 | 105 | 106 | 107 | 108 | 109 | 110 | 111 | 112 |
|-----|-----|-----|-----|-----|-----|-----|-----|-----|-----|
| Phe | Met | Cys | Glu | Tyr | Ala | Asp | Glu | Thr | Ala |
|     | 320 |     |     |     | 330 |     |     | 340 |     |
| TTC | ATG | TGC | GAA | TAC | GCG | GAC | GAA | ACT | GCG |
| AAG | TAC | ACG | CTT | ATG | CGC | CTG | CTT | TGA | CGC |

| 113 | 114 | 115 | 116 | 117 | 118 | 119 | 120 | 121 | 122 |
|-----|-----|-----|-----|-----|-----|-----|-----|-----|-----|
| Thr | Ile | Val | Glu | Phe | Leu | Asn | Arg | Trp | Ile |
|     | 350 |     |     |     | 360 |     |     | 370 |     |
| ACG | ATC | GTT | GAA | TTT | CTG | AAC | CGT | TGG | ATC |
| TGC | TAG | CAA | CTT | AAA | GAC | TTG | GCA | ACC | TAG |

| 123 | 124 | 125 | 126 | 127 | 128 | 129 | 130 | 131 | 132 |
|-----|-----|-----|-----|-----|-----|-----|-----|-----|-----|
| Thr | Phe | Cys | Gln | Ser | Ile | Ile | Ser | Thr | Leu |
|     | 380 |     |     |     | 390 |     |     | 400 |     |
| ACC | TTC | TGC | CAG | TCG | ATC | ATC | TCT | ACC | CTG |
| TGG | AAG | ACG | GTC | AGC | TAG | TAG | AGA | TGG | GAC |

| 133 | 134 | 135 |     |     |     |
|-----|-----|-----|-----|-----|-----|
| Thr | *   |     |     |     |     |
|     | 410 |     |     |     |     |
| ACC | TGA | TAG |     |     | 3' |
| TGG | ACT | ATC | AGC | T   | 5' |

Tabelle 2

DNA-Sequenz II

Sequenz IIa (IL 2-I)

```
                                           ◄───────── Ia ─────────────────►
Nucleotid Nr.                    1                        10
Cod. Strang              5'  AA    TTC    ATG    GCG    CCG
nicht cod. Strang        3'          G    TAC    CGC    GGC
                                    Eco RI    ◄────── Ib ──────────
        ──►     ◄────────── Ic ──────────                         ───►◄─
                20                      30                      40
        ACC    TCT    TCT    TCT    ACC    AAA    AAG    ACT    CAA    CTG
        TGG    AGA    AGA    AGA    TGG    TTT    TTC    TGA    GTT    GAC
        ─────────────────────►  ◄───────── Id ─────────
        ─────────────── Ie ───────────────────────────────────►
                50                      60                      70
        CAA    CTG    GAA    CAC    CTG    CTG    CTG    GAC    CTG    CA
        GTT    GAC    CTT    GTG    GAC    GAC    GAC    CTG    G      PstI
                    ►◄──────────────── If ──────────────────►
```

DNA-Sequenz IIb (IL 2-II)

```
            ◄─────────── IIa ─────────────────────►    ◄───
                80                      90                      100
        PstI   G    ATG    ATC    CTG    AAC    GGT    ATC    AAC    AAC    TAC    AAA
        AC    GTC    TAC    TAG    GAC    TTG    CCA    TAG    TTG    TTG    ATG    TTT
        ◄──────────────── IIb ─────────────────►  ◄──────── IId ───────
        ──────────────── IIc ─────────────────►    ◄─IIe ──────────
                110                     120                     130
        AAC    CCG    AAA    CTG    ACG    CGT    ATG    CTG    ACC    TTC
        TTG    GGC    TTT    GAC    TGC    GCA    TAC    GAC    TGG    AAG
        ◄──────── IId ─────────►  ◄──────────── IIf ───────────
```

```
IIe ─────────────────────→│←──────────── IIg ──────────────────────

         140                          150                        160
AAA    TTC    TAC    ATG    CCG    AAA    AAA    GCT    ACC    GAA
TTT    AAG    ATG    TAC    GGC    TTT    TTT    CGA    TGG    CTT
──────────────→│←───────────────── IIh ─────────────────────────→│

──────────────→│←──── II1 ────────────────→
         170                          180
CTG    AAA    CAC    CTC    CAG    TGT    XbaI
GAC    TTT    GTG    GAG    GTC    ACA    GAT    C
│←──────────────────────── IIj ──────────────────────→
```

DNA-Sequenz IIc (IL 2-III)

```
                                    ───── IIIa ─────
                                           190
                              CTA    GAA    GAA    GAG
                                     TT     CTT    CTC

─────────────────── Xba I ──────────────────→│←──── IIIb ─────────
→│←──── IIIc ─────────────────────────→│←──────── IIIe ──────
         200                          210                        220
CTG    AAA    CCG    CTG    GAG    GAA    GTT  │ CTG    AAC    CTG
GAC    TTT    GGC    GAC    CTC    CTT    OAA    GAC    TTG    GAC
──── IIIb ──────────────→│←──────── IIId ────────────────────→│

────────────────→│←──────── IIIg ────────────→│←─────
         230                          240                        250
GCT    CAG    TCT    AAA    AAT    TTC    CAC    CTG    CGT  │ CCG
CGA    GTC    AGA    TTT    TTA    AAG    GTG    GAC    GCA    GGC
──────── IIIf ───────────────→│←──── IIIh ──────────────────
──────── IIIi ──────────────────────→│←──────────── IIIk─
         260                          270                        280
CGT    GAC    CTG    ATC    TCT    AAC    ATC    AAC    GTT    ATC
GCA    CTG  │ GAC    TAG    AGA    TTG    TAG    TTG    CAA    TAG
←──────────────────── IIIj ────────────────────→│←───
```

IIIk ────────────►

```
        290
GTT   CTG   GAG   CT
CAA   GAC   C    Sac I
```
IIIl ──────────►

DNA-Sequenz II d (IL 2-IV)

◄──────────── IVa ────────────

```
        300                   310
Sac I   C    AAA   GGT   TCT   GAA   ACC   ACG
TC     GAG   TTT   CCA   AGA   CTT   TGG   TGC
```

◄────────────── IV b ──────────────

◄────────────── IVc ──────────────►  ◄

```
        320                   330                   340
TTC   ATG   TGC   GAA   TAC   GCG   GAC   GAA   ACT   GCG
AAG   TAC   ACG   CTT   ATG   CGC   CTG   CTT   TGA   CGC
```
────────────►  ◄──────── IVd ────────

──────────── IVe ────────────►  ◄──────────── IVg

```
        350                   360                   370
ACG   ATC   GTT   GAA   TTT   CTG   AAC   CGT   TGG   ATC
TGC   TAG   CAA   CTT   AAA   GAC   TTG   GCA   ACC   TAG
```
────►  ◄──────── IVf ────────

──────────── IVg ────────►  ◄──────────── IVi ────────

```
        380                   390                   400
ACC   TTC   TGC   CAG   TCG   ATC   ATC   TCT   ACC   CTG
TGG   AAG   ACG   GTC   AGC   TAG   TAG   AGA   TGG   GAC
```
──────────── IVh ────────────►  ◄──────── IVj ────────

IVi ────────────►

```
        410
ACC   TGA   TAG   Sal I        3'
TGG   ACT   ATC   AGC   T       5'
```
IVj ──────────────►

## Patentansprüche

**Patentansprüche für folgende Vertragsstaaten : BE, CH, DE, FR, GB, IT, LI, LU NL und SE**

1. DNA-Sequenz I mit den Nukleotiden 9 bis 407 (Aminosäuren 1 bis 133) gemäß Tabelle 1, codierend für ein Protein mit der biologischen Aktivität des humanen Interleukin-2 (IL-2).

2. DNA-Sequenz I mit den Nukleotiden 6 bis 407 (Aminosäuren 0 bis 133), gemäß Tabelle 1, gefolgt von einem oder zwei Stop-Codon(s), codierend für ein Protein mit der biologischen Aktivität des humanen Interleukin-2 (IL-2).

3. DNA-Sequenzen II a (IL 2-I), II b (IL 2 2-II), II c (IL 2-III) und II d (IL 2-IV) gemäß Tabelle 2.

4. DNA-Oligonukleotide I a bis IV j gemäß Tabelle 2.

5. Hybridplasmide, die zwischen einer Eco RI- und einer Pst I- Schnittstelle die DNA-Sequenz IIa (IL 2-I) oder zwischen einer Pst I- und einer Xba I- Schnittstelle die DNA-Sequenz II b (IL 2-II) oder zwischen einer Xba I-und einer Sac I - Schnittstelle die DNA-Sequenz II c (IL 2-III) oder zwischen einer Sac I- Schnittstelle und einer Sal I-Schnittstelle die DNA-Sequenz II d (IL 2-IV) gemäß Tabelle 2 enthalten.

6. Hybridplasmide, die zwischen einer Eco RI- und einer Sal-I-Schnittstelle die DNA-Sequenz I gemäß Tabelle 1 enthalten.

7. Hybridplasmide, die die DNA-Sequenz I (Nucleotide 6-413) gemäß Tabelle 1 enthalten.

8. Wirtszellen, insbesondere E. coli, die Hybridplasmide nach Anspruch 5 bis 7 enthalten.

9. Gentechnologisches Verfahren zur Herstellung von IL-2, dadurch gekennzeichnet, daß ein synthetisches Gen eingesetzt wird, das die DNA-Sequenz I (Nukleotide 9 bis 407 entsprechend den Aminosäuren 1 bis 133) gemäß Tabelle 1 enthält.

10. Verwendung der DNA-Sequenzen gemäß Tabellen 1 und 2 zur Herstellung abgewandelter DNA-Sequenzen.

**Patentansprüche für folgenden Vertragsstaat : AT**

1. Gentechnologisches Verfahren zur Herstellung von IL-2, dadurch gekennzeichnet, daß ein synthetisches Gen eingesetzt wird, das die DNA-Sequenz I (Nukleotide 9 bis 407 entsprechend den Aminosäuren 1 bis 133) gemäß Tabelle 1 enthält.

2. Verfahren nach Anspruch 1, dadurch gekennzeichnet, daß das Gen die DNA-Sequenz I (Nukleotide 6 bis 407 entsprechend den Aminosäuren 0 bis 133), gemäß Tabelle 1, gefolgt von einem oder zwei Stop-Codon(s), enthält.

3. Verfahren nach Anspruch 1 oder 2, dadurch gekennzeichnet, daß das Gen vor dem Triplett für die erste Aminosäure das Codon ATG aufweist und nach der letzten Aminosäure zwei Stop-Codons aufweist.

4. Verfahren nach Anspruch 1 bis 3, dadurch gekennzeichnet, daß als Wirtsorganismus E. coli dient.

5. Verwendung der DNA-Sequenzen gemäß Tabellen 1 und 2 zur Herstellung abgewandelter DNA-Sequenzen.

**Claims**
**Claims for the following Contracting States : BE, CH, DE, FR, GB, IT, LI, LU, NL and SE**

1. DNA sequence I with the nucleotides 9 to 407 (amino acids 1 to 133) shown in Table 1, coding for a protein with the biological activity of human interleukin-2 (IL-2).

2. DNA sequence I with the nucleotides 6 to 407 (amino acids 0 to 133), shown in Table 1, followed by one or two stop codon(s), coding for a protein with the biological activity of human interleukin-2 (IL-2).

3. DNA sequences IIa (IL 2-I), IIb (IL 2-II), IIc (IL 2-III) and IId (IL 2-IV) shown in Table 2.

4. DNA oligonucleotides Ia to IVj shown in Table 2.

5. Hybrid plasmids which contain between an EcoRI and a PstI cleavage site the DNA sequence IIa (IL 2-I) or between a PstI and an XbaI cleavage site the DNA sequence IIb (IL 2-II) or between an XbaI and a SacI cleavage site the DNA sequence IIc (IL 2-III) or between a SacI cleavage site and a SalI cleavage site the DNA sequence IId (IL 2-IV) shown in Table 2.

6. Hybrid plasmids which contain between an EcoRI and a SalI cleavage site the DNA sequence I shown in Table 1.

7. Hybrid plasmids which contain the DNA sequence I (nucleotides 6-413) shown in Table 1.

8. Host cells, in particular E. coli, which contain hybrid plasmids as claimed in claim 5 to 7.

9. A genetic engineering process for the preparation of IL-2, which comprises employing a synthetic gene which contains the DNA sequence I (nucleotides 9 to 407 corresponding to amino acids 1 to 133) shown in Table 1.

10. The use of the DNA sequences shown in Tables 1 and 2 for preparing modified DNA sequences.

**Claims for the following Contracting State : AT**

1. A genetic engineering process for the preparation of IL-2, which comprises employing a synthetic gene which contains the DNA sequence I (nucleotides 9 to 407 corresponding to amino acids 1 to 133) shown in Table 1.

2. The process as claimed in claim 1, wherein the gene contains the DNA sequence I (nucleotides 6 to 407 corresponding to amino acids 0 to 133), shown in Table 1, followed by one or two stop codon(s).

3. The process as claimed in claim 1 or 2, wherein the gene has, upstream of the triplet for the first amino acid, the codon ATG and, downstream of the last amino acid, two stop codons.

4. The process as claimed in claim 1 to 3, wherein E. coli is used as host organism.

5. The use of the DNA sequences shown in Tables 1 and 2 for preparing modified DNA sequences.

**Revendications**
**Revendications pour les Etats contractants suivants : BE, CH, DE,FR, GB, IT, LI, LU, NL, SE**

1. Séquence d'ADN I comportant les nucléotides 9 à 407 (aminoacides 1 à 133) selon le tableau 1, codant pour une protéine ayant l'activité biologique de l'interleukine 2 (IL-2) humaine.

2. Séquence d'ADN I comportant les nucléotides 6 à 407 (aminoacides 0 à 133) selon le tableau 1, suivie de un ou deux codon(s) d'arrêt, codant pour une protéine ayant l'activité biologique de l'interleukine 2 (IL-2) humaine.

3. Séquences d'ADN IIa (IL 2-I), IIb (IL 2-II), IIc (IL 2-III) et IId (IL 2-IV) selon le tableau 2.

4. Oligonucléotides d'ADN Ia à IVj selon le tableau 2.

5. Plasmides hybrides qui contiennent entre un site de coupure EcoRI et un site de coupure PstI la séquence d'ADN IIa (IL 2-I), ou entre un site de coupure PstI et un site de coupure XbaI la séquence d'ADN IIb (IL 2-II), ou entre un site de coupure XbaI et un site de coupure SacI la séquence d'ADN IIc (IL 2-III) ou entre un site de coupure SacI et un site de coupure SalI la séquence d'ADN IId (IL 2-IV) selon le tableau 2.

6. Plasmides hybrides qui contiennent entre un site de coupure EcoRI et un site de coupure SalI la séquence d'ADN I selon le tableau 1.

7. Plasmides hybrides qui contiennent la séquence d'ADN I (nucléotides 6-413) selon le tableau 1.

8. Cellules hôtes, en particulier E. coli, qui contiennent des plasmides hybrides selon les revendications 5 à 7.

9. Procédé de génie génétique pour la production d'IL-2, caractérisé en ce que l'on utilise un gène synthétique qui contient la séquence d'ADN I (nucléotides 9 à 407 correspondant aux aminoacides 1 à

EP 0 163 249 B1

133) selon le tableau 1.

10. Utilisation des séquences d'ADN selon les tableaux 1 et 2, pour la production de séquences d'ADN modifiées.

**Revendications pour l'Etat contractant suivant : AT**

1. Procédé de génie génétique pour la production d'IL-2, caractérisé en ce que l'on utilise un gène synthétique qui contient la séquence d'ADN I (nucléotides 9 à 407 correspondant aux aminoacides 1 à 133) selon le tableau 1.

2. Procédé selon la revendication 1, caractérisé en ce que la gène contient la séquence d'ADN I (nucléotides 6 à 407 correspondant aux aminoacides 0 à 133) selon le tableau 1, suivie de un ou deux codon(s) d'arrêt.

3. Procédé selon la revendication 1 ou 2, caractérisé en ce que le gène présente le codon ATG avant le triplet codant pour le premier aminoacide, et présente deux codons d'arrêt après le dernier aminoacide.

4. Procédé selon les revendications 1 à 3, caractérisé en ce que l'on utilise E. coli comme organisme hôte.

5. Utilisation des séquences d'ADN selon les tableaux 1 et 2, pour la production de séquences d'ADN modifiées.

16

FIG. 1

FIG.2

FIG.3

FIG.4

FIG.5

FIG. 6